# EUROPEAN PATENT SPECIFICATION

(11) **EP 0 322 746 B1**
(45) Date of publication and mention of the grant of the patent: **29.05.1996**
(21) Application number: 88121439.9
(22) Date of filing: 21.12.1988
(51) Int. Cl.: C07D 471/04, C07D 471/16, C07D 487/04, C07D 487/16, C07D 498/04, C07D 498/16, C07D 513/04, C07D 513/16, A61K 31/335, A61K 31/395

(54) **Heterocyclic compounds**
Heterocyclische Verbindungen
Composés hétérocycliques

(30) Priority: 30.12.1987 US 139539
(43) Date of publication of application: 05.07.1989
(73) Proprietor: ORION CORPORATION LIMITED, SF-02101 Espoo (FI)
(72) Inventor: Sellin, Lawrence, 00970 Helsinki (FI); Bäckström, Reijo Johannes, 00750 Helsinki (FI); Heinola, Kalevi Evert, 04400 Järvenpää (FI); Honkanen, Erkki Juhani, 01400 Vantaa (FI); Langenskiöld, Tord Karl Walter, 02100 Espoo (FI); Ojala, Irma Orvokki, 00660 Helsinki (FI); Pippuri, Aino Kyllikki, 02360 Espoo (FI); Pystynen, Jarmo Johan, 02600 Espoo (FI)
(74) Representative: Weinhold, Peter, Dr.

(56) References cited:
- EP-A- 0 161 632
- EP-A- 0 186 010
- EP-A- 0 189 103
- EP-A- 0 214 592
- EP-A- 0 263 352
- DE-A- 3 410 168
- CHEMICAL ABSTRACTS, vol. 106, no. 13, 30th March 1987, page 649, column 1, abstract no. 102183m, Columbus, Ohio, USA; D.R. SHRUDHAR et al.: "Synthesis and anthelmintic activity of substituted imidazo(4,5-g)-1,4-benzoxazin-3-ones and 6(7)-(acylamino)-7(6)(N,N-bis-(methoxycarbonyl)guanidino)-1,4-benzoxazin-3-ones."

## Description

This invention relates to novel heterocyclic lactam compounds and salts thereof useful as cardiotonic agents, antihypertensive agents and vasodilatators for the treatment of congestive heart failure. This invention also relates to processes for preparing the same as well as pharmaceutical compositions comprising these compounds or salts thereof.

EP-A-0 214 592 discloses compounds with cardiotonic activity having the general formula 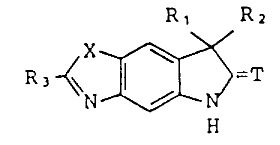 wherein, for instance,
- R₁ and R₂: are independently hydrogen or an alkyl group,
- T: is oxygen,
- X: is sulfur, and
- R₃: is hydrogen or an amino group.

EP-A-0 161 632 discloses compounds with cardiotonic activity having the general formula 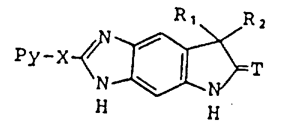 wherein, for instance,
- R₁ and R₂: independently are hydrogen or an alkyl group,
- X: is a valence bond or a C₁-C₄ alkylene group,
- T: is oxygen, and
- Py: is an optionally substituted 2-, 3- or 4-pyridyl residue.

EP-A-0 189 103 discloses compounds with cardiotonic activity having the general formula 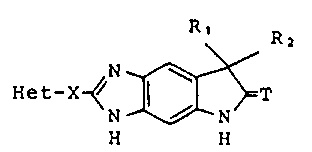 wherein, for instance,
- R₁ and R₂: independently are hydrogen or an alkyl group,
- X: is a valence bond or a C₁-C₄ alkylene group,
- T: is oxygen, and
- Het: represents a heterocyclic 6-membered ring having an oxygen or a sulfur atom, a heterocyclic 5-membered ring having 1-4 heteroatoms or a heterocyclic 6-membered ring having 2-5 heteroatoms, whereby the heteroatoms of said 5- and 6-membered rings may be the same or different from each other and are nitrogen, oxygen or sulfur.

The novel compounds according to the present invention useful as inotropic agents are heterocyclic lactam compounds of formula I or II: 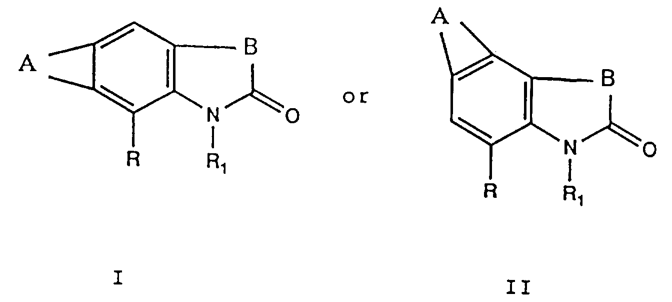 and phamaceutically acceptable salts thereof, in which A is -N=N-NH-, -S-C(R₂)=N- or -N=C(R₂)-S-, wherein R₂ is amino, pyridyl, pyrimidinyl, aminopyrimidinyl, oxidopyridyl, morpholinyl or trimethoxyphenyl, B is -(CH₂)ₘ-C(R₃R₄)-(CH₂)ₙ, wherein R₃ and R₄ are independently hydrogen or C₁₋₆-alkyl and m and n are different from each other and are 0 or 1, R is hydrogen, C₁₋₆-alkyl, amino or halogen, R₁ is hydrogen or C₁₋₆-alkyl or R and R₁ together form a -CH=N-CH₂- group.

In the foregoing definitions, the C₁₋₆-alkyl moieties are preferably represented by methyl, ethyl, straight-chain propyl, butyl, pentyl or hexyl. Most preferred is methyl.

Congestive heart failure is characterized by a decrease in cardiac output and an increase in right and left ventricular filling pressure. This hemodynamic condition can produce symptoms of dyspnoea, fatigue and edema. Treatment of heart disease usually focuses on the three principle factors determining cardiac performance: preload, impedance and an increase in systolic performance (contractility). Vasodilation can improve cardiac function by reducing preload and impedance. Cardiac output can be enhanced by augmenting contractility. This is the rationale for developing inotropic compounds.

In the present invention the preferred structures of new compounds which have particular usefulness as inotropic agents are presented by the following formulas: 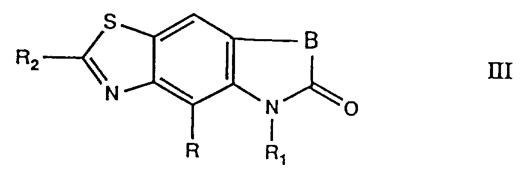 and 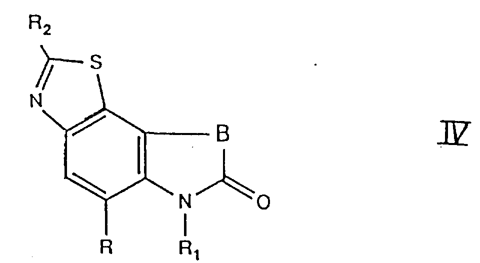 wherein R₂ is amino, pyridyl, pyrimidinyl, aminopyrimidinyl, oxidopyridyl, morpholinyl or trimethoxyphenyl, B is -(CR₂)ₘ-C(R₃R₄)-(CH₂)ₙ, wherein R₃ and R₄ are independently hydrogen or C₁₋₆-alkyl and m and n are different from each other and are 0 or 1, R is hydrogen, C₁₋₆-alkyl, amino or halogen, R₁ is hydrogen or C₁₋₆-alkyl or R and R₁ together form a -CH=N-CH₂- group.

Particularly preferred are compounds of formulas III or IV, wherein R₂ is pyridyl, R is hydrogen or halogen, R₃ and R₄ are independently hydrogen or methyl, R₁ is hydrogen or R and R₁ together form a -CH=N-CH₂- group.

The compounds of Formula I and II may be prepared in accordance with the following reaction sequences.

The bicyclic portion of the molecule containing the lactam ring 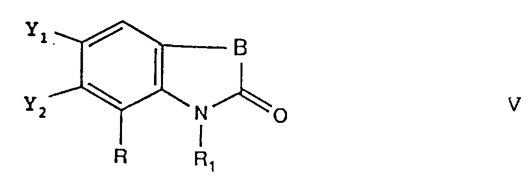 wherein B, R and R₁ are as defined above and Y₁ is hydrogen, -OH, -SH or -NH₂ when Y₂ is -NH₂ or Y₂ is hydrogen, -OH, -SH or -NH₂ when Y₁ is -NH₂, can be prepared according to methods known in the literature.

Compound V, in the case wherein Y₁ is hydrogen and Y₂ is amino or contrary Y₁ is amino and Y₂ is hydrogen, is reacted in the presence of sulphur with a compound having the formula VI,

R₂ - CH₃ VI

wherein R₂ is as defined above,
to give the compounds I or II according to the invention, wherein A is -N=C(R₂)-S- or -S-C(R₂)=N- and R₂ is as defined above.

The compounds I or II wherein A is -N=N-NH- may be prepared from V wherein Y₁ and Y₂ are -NH₂ by treating with nitrous acid.

### Determination of inotropic activity

The inotropic activity has been determined by the aid of the method described below.

Male or female guinea-pigs (300-400 g) of the Dunkin-Hartley strain were killed by a sharp blow to the head. Their hearts were immediately excised and placed in an oxygenated (95% O₂ - 5%CO₂) Krebs-Ringer solution having the following composition (mM): NaCl, 135; KCl, 5; MgCl₂,1; CaCl₂,2; Na₂HPO₄, 1; NaHCO₃, 15; glucose 10 (pH 7.2 - 7.4). Right ventricular papillary muscles were dissected out and placed in a 5 ml - temperature-regulated (30±1°C) organ bath and continuously suffused with the Krebs-Ringer solution at a rate of 6±1 ml min⁻¹. One end of each papillary muscle was fixed to the chamber with syringe needles (26 gauge), while the other was connected via a hooked metal rod to an isometric force transducer (FT.03, Grass Instruments, Quincy, Massachusetts, U.S.A.). The muscles were stretched to a resting tension of 300 mg and were stimulated with bipolar silver electrodes at a rate of 0.5 Hz using single square pulses (5-10V, 0.5-1.0 msec) from an analog stimulator (Model S88, Grass) coupled to a constant current (1-10 mA) unit (model PSIU6, Grass). After an equilibration period of 30-45 min, the muscles were consecutively exposed, for 10 min each, to 0.4 % DMSO alone and test compounds concentrations of 1, 3, 10 and 30 µM with 0.4 % DMSO as the vehicle.

Isometric tension was measured via a low-level D.C. amplifier and a D.C. driver amplifier (Models 7P1F and 7DAG, respectively, Grass). The output was fed into a programmable digitizer (Model 390AD, Tektronix, Beaverton, Oregon, U.S.A.) and connected, via a IEEE-488 bus line and interface to a Professional 380 (PDP-11/70) microcomputer (Digital Eguipment Corp., Maynard, Massachusetts, U.S.A.). Data acquisition and analysis was done in real-time using a PASCAL program.

The inotropic response for the test compounds at a concentration of 10 µM is shown in Table 1.

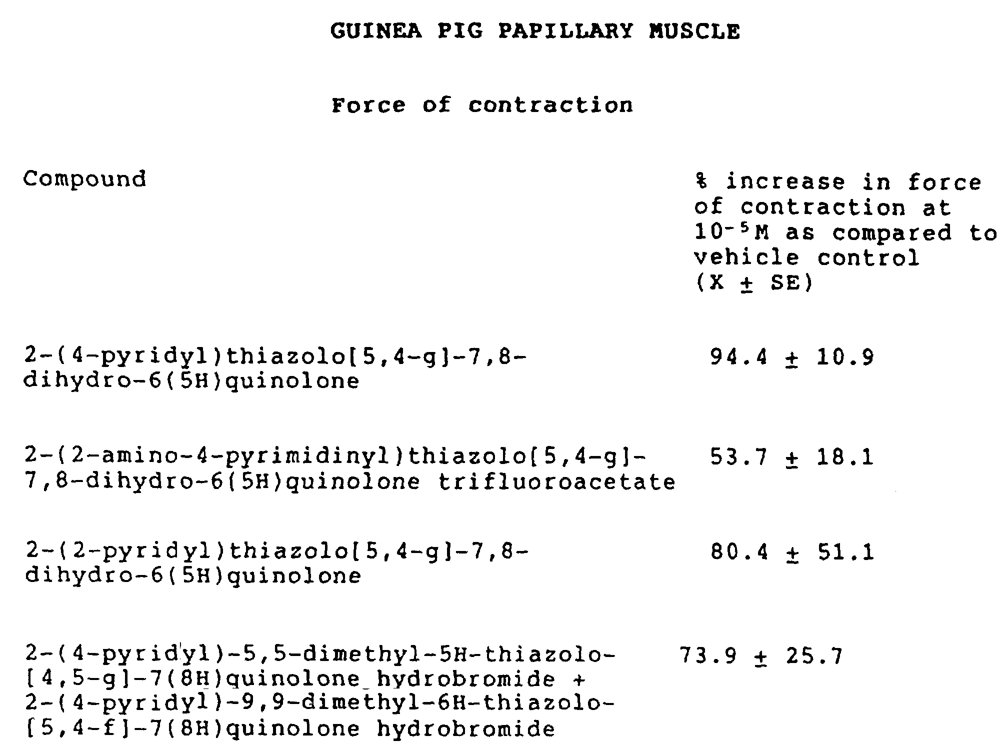

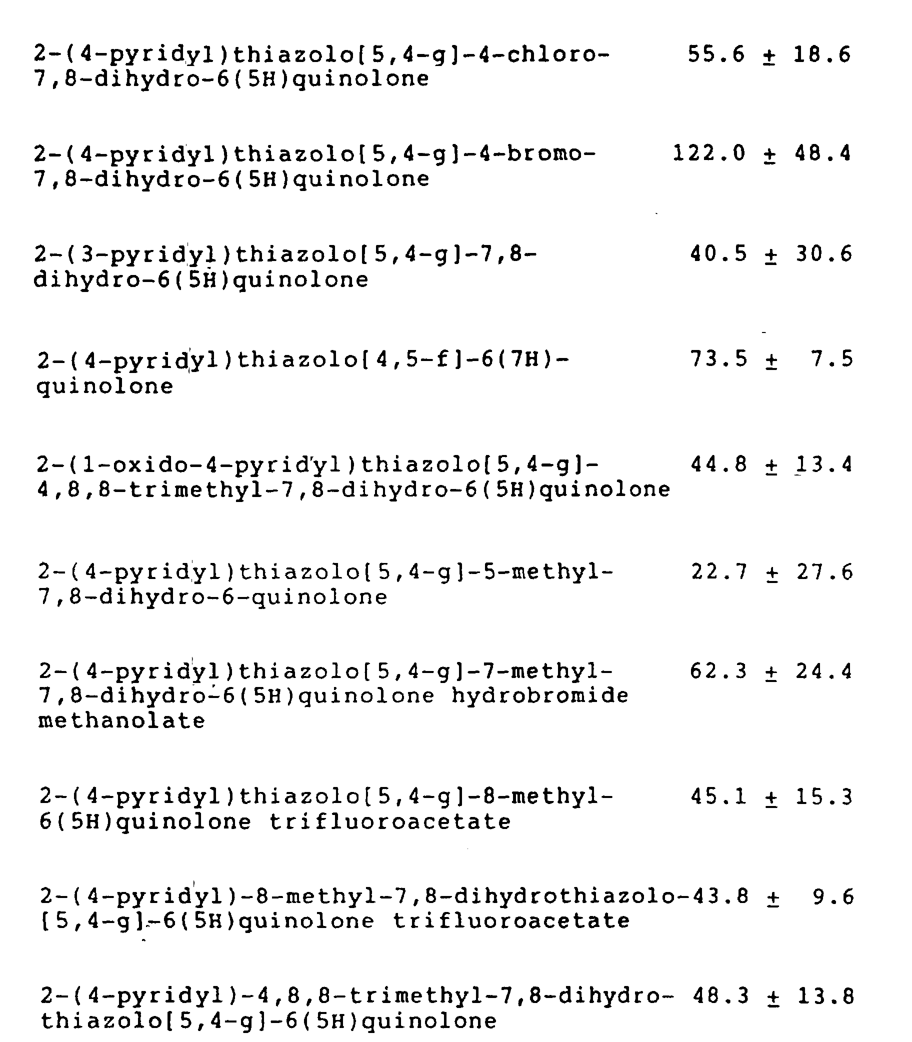

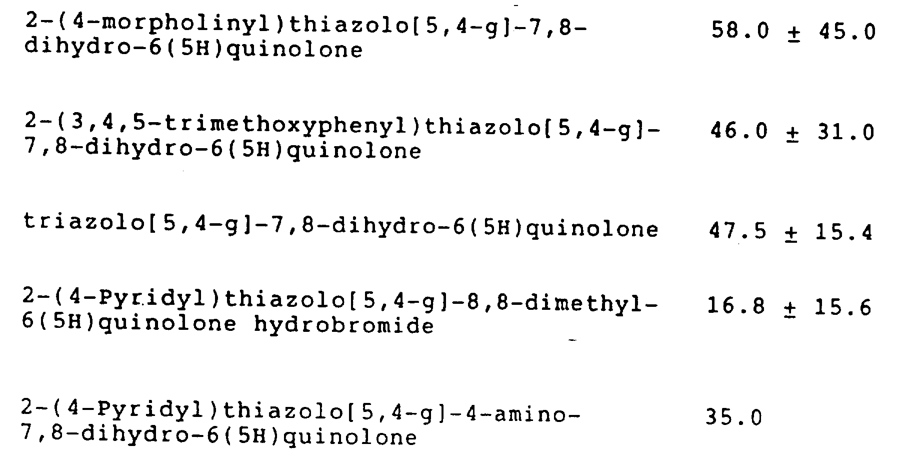

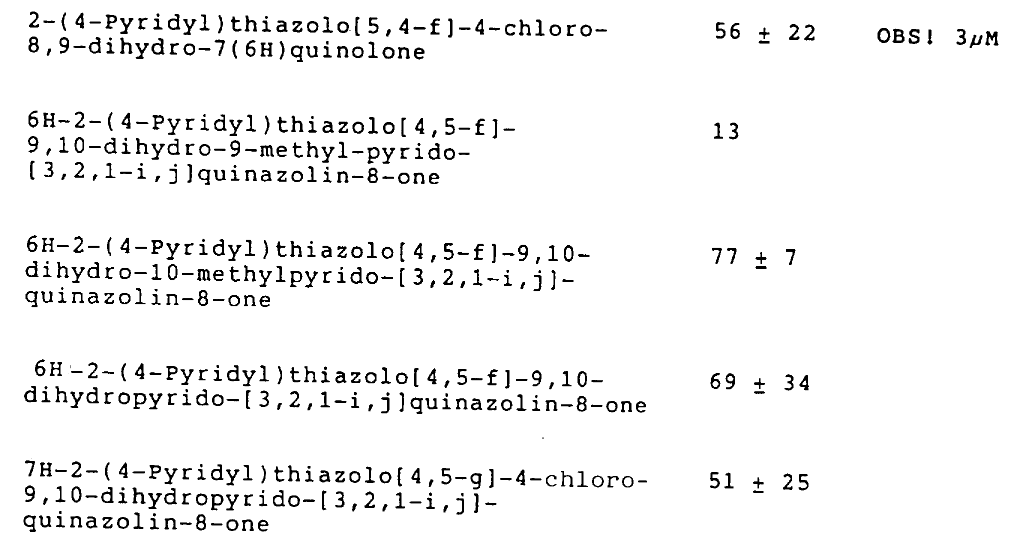

The following examples illustrate the invention in greater detail

### Example 1

### 2-(4-Pyridyl)thiazolo[5,4-g]-7,8-dihydro-6(5H)quinolone

A mixture containing 3,6 g of 7-amino-3,4-dihydro-2(1H)quinolone and 2,5 g of sulfur in 7,5 ml of 4-picoline was heated for 5-6 h at 160°C. After cooling the mixture was filtered, washed first with small amount of 4-picoline and then with ether. The crude product was recrystallized from DMF. Yield 5,0 g (80 %), m.p. 309-314°C.

### Example 2

### 2-(2-Pyridyl)thiazolo[5,4-g]-7,8-dihydro-6(5H)quinolone

The procedure described in Example 1 was repeated by using 2-picoline. Yield 2,1 g (33 %), m.p. 246-247°C.

### Example 3

### 2-(2-Amino-4-pyrimidinyl)thiazolo[5,4-g]-7,8-dihydro-6(5H)quinolone trifluoroacetate

The procedure described in Example 1 was repeated by using 1,2 g of 7-amino-3,4-dihydro-2(1H)quinolone, 0,83 g of sulfur and 1,61 g of 2-amino-4-methylpyrimidine in 2,5 ml of DMA. After cooling the mixture was diluted with methanol and filtered. The crude product was dissolved in boiling trifluoroacetic acid, filtered and diluted with 2-propanol. Yield 0,3 g (10 %), m.p. 350°C (decomp.).

### Example 4

### 7-Amino-6-mercapto-3,4-dihydro-2(1H)quinolone hydrochloride

To a solution containing 11,0 g of 3-chloro-4,6-dinitrophenylpropionic acid in 100 ml of ethanol, 5,6 g of sodiumsulfide nonahydrate and 0,8 g of sulphur in 5 ml of water were added. The solution was stirred for 1 h at 60°C and cooled. The intermediate product, bis(3-carboxyethyl-4,6-dinitrophenyl) disulfide, was filtered, washed with water and dissolved in 150 ml of 90 % acetic acid. 15,0 g of zinc dust was gradually added while stirring at room temperature. The mixture was then refluxed for 30 min and filtered. The filtrate was saturated with gaseous hydrogen chloride, concentrated in vacuo and ether was added. The product was filtered. Yield 4,8 g, m.p. 177°C.

### Example 5

### 2-(3-pyridyl)thiazolo[5,4-g]-7,8-dihydro-6(5H)quinolone

A mixture containing 2,31 g of 7-amino-6-mercapto-3,4-dihydro-quinolone and 1,8 g of nicotinic acid in 15 ml of polyphosphonic acid, was heated for 2 h at 100-120°C. After cooling water was added and the product was filtered and washed with water. Yield 65%.

### Example 6

### 2-(4-Pyridinyl)thiazolo[5,4-g]-7-methyl-7,8-dihydro-6(5H)quinolone

The procedure described in Example 1 was repeated by using 4 ml of 4-picoline, 1,8 g of sulphur and 1,8 g of 7-amino-3-methyl-3,4-dihydro-2(1H)quinolone. Yield 1,2 g, m.p. 287-292°C.

### Example 7

### A mixture of 2-(4-Pyridyl)-5,5-dimethyl-5H-thiazolo[4,5-g]-7(8H)quinolone hydrobromide and 2-(4-pyridyl)-9,9-dimethyl-6H-thiazolo[5,4-f]-7(8H)quinolone hydrobromide.

A mixture containing 3 ml of 4-picoline, 0.5 g of sulfur and 0.82 g of 6-amino-4,4-dimethyl-3,4-dihydro-2(1H)quinolone was heated for 5 - 6 h at 160°C to form the intermediate compound, 6-(4-pyridylthiocarbonylamino)-4,4-dimethyl-3,4-dihydro-2(1H)quinolone, which is dissolved in 20 ml DMF. 0.16 ml bromine was added and the solution was heated for 20 min at 100 - 120°C. After cooling the product was filtered, washed with DMF and acetone to give the title compounds. Mp. 216-222°C.

### Example 8

### 2-(4-Pyridyl)-8-methyl-7,8-dihydrothiazolo[5,4-g]-6(5H)quinolone trifluoroacetate

The procedure described in Example 1 was repeated by using 7-amino-4-methyl-2(1H)-quinolone and 4-picoline as starting materials. Mp. 275-280°C.

### Example 9

### 2-(4-Pyridyl)-4,8,8-trimethyl-7,8-dihydrothiazolo[5,4-g]6(5H)quinolone

The method described in Example 1 was repeated by using 7-amino-4,4,8-trimethyl-2(1H)quinolone as starting material. Mp. 305-307°C.

### Example 10

### 2-(4-Morpholino)thiazolo[5,4-g]-7,8-dihydro-6(5H)quinolone

A solution containing 0,2 g of 2-chlorothiazolo[5,4-g]-7,8-dihydro-6(5H)quinolone in 5 ml of morpholine was refluxed for 5 h. The express of morpholine was evaporated in vacuo and the residue was treated water. The crystals were filtered and washed with ether, yield 0,1 g, m.p. 275°C.

### Example 11

### 2-(4-Pyridyl)thiazolo[5,4-g]-8-methyl-6(5H)quinolone trifluoroacetate

A mixture containing 1,17 g of 7-amino-4-methyl-2(1H)quinolone, 0,75 g of sulphur and 3,0 ml of 4-picoline was heated for 7 h at 160°C. The reaction mixture was treated with methanol and the product was filtered, washed with methanol and dissolved in DMF. 1 ml of sulphur monochloride was added and the mixture was stirred for 15 min at room temperature. The product was filtered and washed with DMF and carbondisulphide and triturated then with trifluoroacetic acid. Sulphur was filtered off and to the filtrate 2-propanol was added. The solution was concentrate in vacuo, the crystals were filtered and washed with 2-propanol, m.p. >350°C.

### Example 12

### 2-(4-Pyridyl)thiazolo[5,4-g]-4-bromo-7,8-dihydro-6(5H)quinolone

The compound described in Example 1 was treated with bromine in DMF at room temperature. Dil. ammonium hydroxide solution was then added. The product was filtered and washed with water, m.p. 299-300°C.

### Example 13

### 2-(4-Pyridyl)thiazolo[5,4-g]-4-chloro-7,8-dihydro-6(5H) quinolone

To a solution containing 0,42 g of the compound described in Example 1 in 10 ml of DMF, 0,2 g of N-chlorosuccinimide and catalytic amount of 3-chloroperoxybenzoic acid were added. The mixture was heated for 5 min at 150°C. After cooling to 70°C the product was filtered and washed with DMF and acetone. Yield 0,35 g, m.p. 330-333°C.

### Example 14

### 2-(4-Pyridyl)thiazolo[5,4-g]-4-nitro-7,8-dihydro-6(5H)quinolone

To a solution containing 0,56 g of the compound described in Example 1 in 5 ml conc. sulphuric acid an equivalent amount of conc. nitric acid was added at room temperature. The solution was stirred for 4 h at 20°C and poured the to ice-water. The solution was neutralized with NH₄OH. The product was filtered and washed with water. Yield 0,58 g, 284-289°C.

### Example 15

### 2-(4-Pyridyl)thiazolo[5,4-g]-4-amino-7,8-dihydro-6(5H)quinolone

The compound obtained in Example 14 was dissolved in conc. hydrochloric acid and treated with stannous chloride solution in conc. hydrochloric acid. The mixture was stirred for 45 min at room temperature. The product was filtered and washed with conc. hydrochloric acid. The free base was liberated in boiling NaOH-solution. The product was filtered and washed with water, m.p. 341-346°C.

### Example 16

### 2-(3,4,5,-Trimethoxyphenyl)thiazolo[5,4-g]-7,8-dihydro-6(5H)quinolone

A mixture containing 0,32 g of 7-amino-3,4-dihydro-2(1H)quinolone, 0,39 g of 3,4,5-trimethoxybenzaldehyde and 0.13 g of sulphur in 2 ml of DMF was stirred for 6 h at 160°C. The reaction mixture was filtered and washed with DMF and ether, Yield 0,3 g, m.p. 289-292°C.

### Example 17

### 2-(4-Pyridyl)thiazolo[5,4-f]-4-chloro-8,9-dihydro-7(6H)quinolone

A mixture containing 0,5 g of 6-amino-7-chloro-3,4-dihydro-2(1H)quinolone, 0,3 g of pyridine-4-carboxaldehyde and 0,16 g of sulphur in 2 ml of DMA was heated for 2 h at 170°C. After cooling 15 ml of methanol was added and sulphur was filtered off. To the filtrate 0,15 ml of bromine was added. The precipitate was filtered and washed with methanol. Yield 0,35 g, m.p. >350°C.

### Example 18

### 2-(4-Pyridyl)thiazolo[4,5-f]-7(6H)quinolone

A mixture containing 0,4 g of 5-amino-2(1H)quinolone 3,0 ml of 4-picoline and 0,32 g of sulphur was heated for 6 h at 160°C. The mixture was filtered and the precipitate was dissolved in DMF after which 0,035 ml of bromine was added. The solution was stirred for 1 h at room temperature, filtered and the product washed with DMF and acetone. Yield 0,15 g, m.p. >350°C.

### Example 19

### Triazolo[5,4-g]-7,8-dihydro-6(5H)quinolone

To solution containing 1,4 g 6-amino-3-methyl-7-(4-pyridinethiocarboxamino)-2(1H)quinolone in 50 ml lmol hydrochloric acid 4,0 g of sodium nitrite. was gradually added with stirring and cooling at 0-5°C. The solution was warmed to room temperature and made first basic with NaOH and then acidic with hydrochloric acid. Finally the pH was adjusted to 8.0 with NaHCO₃. The solution was extracted with ethyl acetate. The solvent was evaporated in vacuo and the residue was crystallized from acetone-dichloromethane by adding petroleum ether. Yield 0,3 g, mp 155°C (decomp.)

### Example 20

### 2-(4-Pyridyl)thiazolo[5,4-g]-7-methyl-7,8-dihydro-6(5H)quinolone hydrobromide methanolate

A mixture of 1,4 g of 7-amino-3-methyl-2(1H)quinolone, 4 ml of 4-picoline and 1-2 g of sulphur was heated for 4 h at 160°C. To the reaction mixture 20 ml of MeOH was added and the precipitate was filtered. The intermediate product was dissolved in DMF and 0,2 ml of bromine was added. The mixture was stirred for 2 h at room temperature, diluted with ether and filtered. Yield 1,2 g, m.p. 287-292°C.

### Example 21

### 2-(4-Pyridyl)thiazolo[5,4-f]-4-chloro-5,9,9-trimethyl-8,9-dihydro-7(6H)quinolone trifluoroacetate

A mixture containing 1,2 g of 6-amino-7-chloro-4,4,8-trimethy-1-2(1H)quinolone and 0,48 g sulphur in 5 ml of 4-picoline was refluxed for 2,5 h. The excess of 4-picoline was evaporated in vacuo. The residue was dissolved in 25 ml of DMF and treated with 0,26 ml of bromine under cooling (0°C). The mixture was heated for 10 min at 100°C, cooled to 0°C. The precipitate was filtered and washed with DMF and acetone. The product was suspended in NaHCO₃ solution,- filtered and washed with water. The crude free base was dissolved in DMSO and treated with trifluoroacetic acid and the product was filtered and washed with DMSO and acetone, m.p. 335-340°C.

### Example 22

### 2-(4-Pyridyl)thiazolo[5,4-f]-4-chloro-7(6H)quinolone

To a solution containing 0,78 g of the product obtained in Example 17 was dissolved in 5 ml of conc. H₂SO₄ 0,14 ml of conc. nitric acid was added with stirring for 24 h at 50°C. The reaction mixture was poured in cold water and washed with NaHCO₃ solution and water, m.p. >350°C.

### Example 23

### 2-(4-Pyridyl)thiazolo[5,4-g]-8,8-dimethyl-6(5H)quinolone hydrobromide

To a solution containing 1,0 g of 4,4-dimethyl-7-(4-pyridinethiocarboxamido)-3,4-dihydro-2(1H)quinolone in 5 ml of pyridine 0,39 ml of sulphur monochloride was added. the mixture was stirred for 30 min at room temperature. The precipitate was filtered and washed with carbon disulfide and acetone. Yield 0,1 g, m.p. 294-295°C.

### Example 24

### 2-(4-Pyridyl)thiazolo[5,4-g]-7,8-dihydro-5-methyl-6-quinolone

To a mixture containing 1,8 g of the product obtained in Example 1 in 20 ml of DMF 0,51 g of sodium hybride dispersion in mineral oil (50 %) was added with stirring at room temperarure. After 2 h 1 ml of methyl iodide was added and the mixture was stirred further for 20 min at 20°C. 150 ml of dichloromethane was then added. The solution was washed with dil. NH₄OH. The solvent was evaporated in vacuo and the residue triturated with acetone and filtered. Yield 0,43 g, m.p. 216-222°C.

### Example 25

### 2-Aminothiazolo[5,4-g]-7,8-dihydro-6(5H)quinolone

To a solution containing 5,0 g of 7-amino-2(1H)quinolone and 5,25 g of sodium thiocyanate in 25 ml of anhydrous acetic acid 1,5 ml of bromine was gradually added with stirring at room temperature. The mixture was stirred 15 min more and filtered. The product was dissolved in 6 mol hydrochloric acid heated for 30 min at 100°C. The pH was then adjusted 7,00 with sodium hydroxide solution. The precipitate was filtered and washed with water and acetone. Yield 1,86 g, m.p. >250°C (decomp).

### Example 26

### 6H-2-(4-Pyridyl)thiazolo[4,5-f]-9,10-dihydropyrido-[3,2,1-i,j]quinazolin-8-one

A mixture containing 1,0 g 2-(4-Pyridyl)thiazolo[4,5-g]-7,8-dihydro-6(5H)quinolone 1,0 g hexamethylenetetramine in 10 ml of trifluoroacetic acid was refluxed for 2 h. To the boiling solution 7 ml of water was added and the solution was evaporated to dryness. To the residue 10 ml of water was added and the pH was adjusted to 5 with NaOH-solution. The product was filtered and washed with water. Yield 46%, m.p. 273-275°C. using 2-(4-pyridyl)thiazolo[5,4-g]-7,8-dihydro-6(5H)quinolone as starting material. Yield 46 %, m.p. 273-275°C.

### Example 27

### 6H-2-(4-Pyridyl)thiazolo[4,5-f]-9,10-dihydro-10-methylpyrido[3,2,1-i,j]quinazolin-8-one

The process described in Example 26 was repeated by using 2-(4-pyridyl)thiazolo[5,4-g]-7,8-dihydro-8-methyl-6(5H)quinolone as starting material. Yield 40 %, m.p. 266-271°C.

### Example 28

### 6H-2-(4-Pyridyl)thiazolo[4,5-f]-9,10-dihydro-9-methylpyrido[3,2,1-i,j]quinazolin-8-one

The process described in Example 26 was repeated by using 2-(4-pyridyl)thiazolo[5,4-g]-7,8-dihydro-7-methyl-6(5H)quinolone as starting material. Yield 27 %, m.p. 247-251°C.

### Example 29

### 7H-2-(4-Pyridyl)thiazolo[4,5-g]-4-chloro-9,10-dihydropyrido[3,2,1-i,j]quinazolin-8-one

The process described in Example 26 was repeated by using 2-(4-pyridyl)thiazolo[5,4-f]-4-chloro-7,8-dihydro-6(5H)quinolone as starting material. Yield 33 %, m.p. 276-280°C.

Preferred pharmaceutically acceptable salts are halides particularly (hydro)chlorides, (hydro)bromides and (hydro)-iodides, and (trifluoro)acetates.

## Claims

1. Compounds of the formula 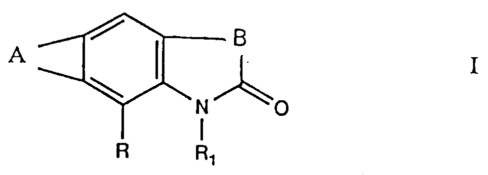 and phamaceutically acceptable salts thereof, in which A is -N=N-NH-, -S-C(R₂)=N- or -N=C(R₂)-S-, wherein R₂ is amino, pyridyl, pyrimidinyl, aminopyrimidinyl, oxidopyridyl, morpholinyl or trimethoxyphenyl, B is -(CH₂)ₘ-C(R₃R₄) - (CH₂)ₙ-_{,} wherein R₃ and R₄ are independently hydrogen or C₁₋₆-alkyl and m and n are different from each other and are 0 or 1, R is hydrogen, C₁₋₆-alkyl, amino or halogen, R₁ is hydrogen or C₁₋₆-alkyl or R and R₁ together form a -CH=N-CH₂- group.

2. Compounds of the formula 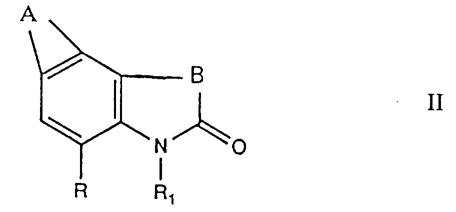 and phamaceutically acceptable salts thereof, in which A is -N=N-NH-, -S-C(R₂)=N- or -N=C(R₂)-S-, wherein R₂ is amino, pyridyl, pyrimidinyl, aminopyrimidinyl, oxidopyridyl, morpholinyl or trimethoxyphenyl, B is -(CH₂)ₘ-C(R₃R₄)-(CH₂)ₙ, wherein R₃ and R₄ are independently hydrogen or C₁₋₆-alkyl and m and n are different from each other and are 0 or 1, R is hydrogen, C₁₋₆-alkyl, amino or halogen, R₁ is hydrogen or C₁₋₆-alkyl or R and R₁ together form a -CH=N-CH₂- group.

3. Compound according to claim 1 having the formula 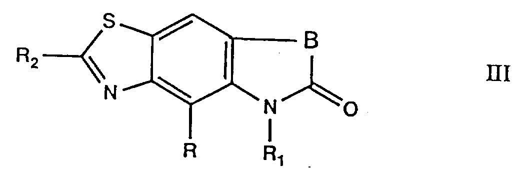 wherein R₂ is amino, pyridyl, pyrimidinyl, aminopyrimidinyl, oxidopyridyl, morpholinyl or trimethoxyphenyl, B is -(CH₂)ₘ-C(R₃R₄) - (CH₂)ₙ-, wherein R₃ and R₄ are independently hydrogen or C₁₋₆-alkyl and m and n are different from each other and are 0 or 1, R is hydrogen, C₁₋₆-alkyl, amino or halogen, R₁ is hydrogen or C₁₋₆-alkyl or R and R₁ together form a -CH=N-CH₂- group.

4. Compound according to claim 2 having the formula 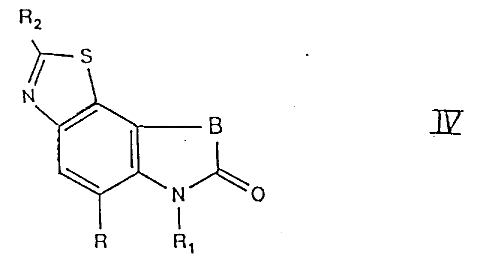 wherein R₂ is amino, pyridyl, pyrimidinyl, aminopyrimidinyl, oxidopyridyl, morpholinyl or trimethoxyphenyl, B is -(CH₂)ₘ-C(R₃R₄)-(CH₂)ₙ-, wherein R₃ and R₄ are independently hydrogen or C₁₋₆-alkyl and m and n are different from each other and are 0 or 1, R is hydrogen, C₁₋₆-alkyl, amino or halogen, R₁ is hydrogen or C₁₋₆-alkyl or R and R₁ together form a -CH=N-CH₂- group.

5. Compound according to claim 3 or 4, wherein R₂ is pyridyl, R is hydrogen or halogen, R₃ and R₄ are independently hydrogen or methyl, R₁ is hydrogen or R and R₁ together form a -CH=N-CH₂- group.

6. Compound of any of claims 1 to 5 which is
2-(4-pyridyl)thiazolo[5,4-g]-4-chloro-7,8-dihydro-6(5H)quinolone,
2-(4-pyridyl)thiazolo[5,4-g]-4-bromo-7,8-dihydro-6(5H)quinolone,
2-(4-pyridyl)thiazolo[4,5-f]-6(7H)quinolone,
2-(1-oxido-4-pyridyl)thiazolo[5,4-g]-4,8,8-trimethyl-7,8-dihydro-6(5H)quinolone,
2-(3-pyridyl)thiazolo[5,4-g]-7,8-dihydro-6(5H)quinolone,
2-(4-pyridyl)thiazolo[5,4-g]-5-methyl-7,8-dihydro-6(5H)quinolone,
2-(4-pyridyl)thiazolo[5,4-g]-8-methyl-6(5H)quinolone trifluoroacetate,
2-(4-pyridyl)thiazolo[5,4-g]-7-methyl-7,8-dihydro-6(5H)quinolone hydrobromide methanolate,
2-(4-pyridyl)-5,5-dimethyl-5H-thiazolo[4,5-g]-7(8H)quinolone hydrobromide,
2-(4-pyridyl)-9,9-dimethyl-6H-thiazolo[5,4-f]-7(8H)quinolone hydrobromide,
2-(4-pyridyl)-8-methyl-7,8-dihydrothiazolo[5,4-g]-6(5H)-quinolone trifluoroacetate,
2-(4-pyridyl)-4,8,8-trimethyl-7,8-dihydrothiazolo[5,4-g]-6(5H)quinolone,
2-(2-pyridyl)thiazolo[5,4-g]-7,8-dihydro-6(5H)quinolone,
2-(2-amino-4-pyrimidinyl)thiazolo[5,4-g]-7,8-dihydro-6(5H)quinolone trifluoroacetate,
2-(4-morpholinyl)thiazolo[5,4-g]-7,8-dihydro-6(5H)quinolone,
2-(3,4,5-trimethoxyphenyl)thiazolo[5,4-g]-7,8-dihydro-6(5H)quinolone,
triazolo[5,4-g]-7,8-dihydro-6(5H)quinolone or
2-(4-pyridyl)thiazolo[5,4-g]-7,8-dihydro-6(5H)quinolone.

7. A composition for the treatment of congestive heart failure, said composition comprising a pharmaceutically acceptable inert carrier and, as the active component thereof, an effective amount of a compound as defined in anyone of claims 1 to 6.

## Patentansprüche

1. Verbindungen der Formel 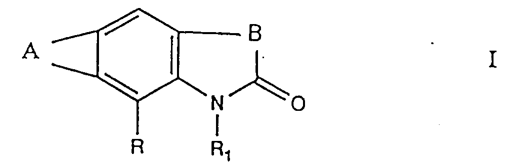 und pharmazeutisch verwendbare Salze davon, wobei A = -N=N-NH-, -S-C(R₂)=N- oder -N=C(R₂)-S- ist, worin R₂ Amino, Pyridyl, Pyrimidinyl, Aminopyrimidinyl, Oxidopyridyl, Morpholinyl oder Trimethoxyphenyl bedeutet,
B = -(CH₂)ₘ-C(R₃R₄)-(CH₂)ₙ- ist, worin R₃ und R₄ unabhängig voneinander Wasserstoff oder C₁₋₆-Alkyl bedeuten und m und n voneinander verschieden sind und 0 oder 1 bedeuten, R = Wasserstoff, C₁₋₆-Alkyl, Amino oder Halogen ist, R₁ Wasserstoff oder C₁₋₆-Alkyl ist oder R und R₁ zusammen eine -CH=N-CH₂-Gruppe bilden.

2. Verbindungen der Formel 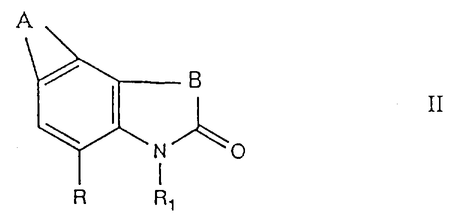 und pharmazeutisch verwendbare Salze davon,
wobei A = -N=N-NH-, -S-C(R₂)=N- oder -N=C(R₂)-S- ist, worin R₂ Amino, Pyridyl, Pyrimidinyl, Aminopyrimidinyl, Oxidopyridyl, Morpholinyl oder Trimethoxyphenyl bedeutet,
B = -(CH₂)ₘ-C(R₃R₄)-(CH₂)ₙ- ist, worin R₃ und R₄ unabhängig voneinander Wasserstoff oder C₁₋₆-Alkyl bedeuten und m und n voneinander verschieden sind und 0 oder 1 bedeuten, R = Wasserstoff, C₁₋₆-Alkyl, Amino oder Halogen ist, R₁ Wasserstoff oder C₁₋₆-Alkyl ist oder R und R₁ zusammen eine -CH=N-CH₂-Gruppe bilden.

3. Verbindung nach Anspruch 1 mit der Formel 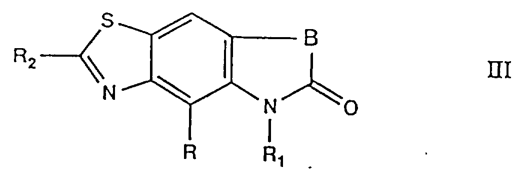 wobei R₂ = Amino, Pyridyl, Pyrimidinyl, Aminopyrimidinyl, Oxidopyridyl, Morpholinyl oder Trimethoxyphenyl bedeutet,
B = -(CH₂)ₘ-C(R₃R₄)-(CH₂)ₙ- ist, worin R₃ und R₄ unabhängig voneinander Wasserstoff oder C₁₋₆-Alkyl bedeuten und m und n voneinander verschieden sind und 0 oder 1 bedeuten, R = Wasserstoff, C₁₋₆-Alkyl, Amino oder Halogen ist, R₁ Wasserstoff oder C₁₋₆-Alkyl ist oder R und R₁ zusammen eine -CH=N-CH₂-Gruppe bilden.

4. Verbindung nach Anspruch 2 mit der Formel 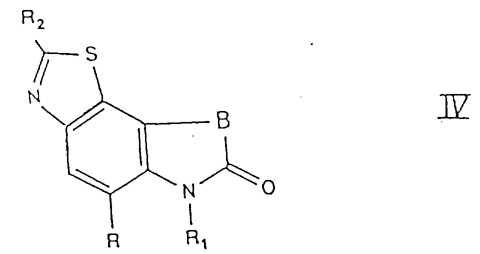 wobei R₂ Amino, Pyridyl, Pyrimidinyl, Aminopyrimidinyl, Oxidopyridyl, Morpholinyl oder Trimethoxyphenyl bedeutet,
B = -(CH₂)ₘ-C(R₃R₄)-(CH₂)ₙ- ist, worin R₃ und R₄ unabhängig voneinander Wasserstoff oder C₁₋₆-Alkyl bedeuten und m und n voneinander verschieden sind und 0 oder 1 bedeuten, R = Wasserstoff, C₁₋₆-Alkyl, Amino oder Halogen ist, R₁ Wasserstoff oder C₁₋₆-Alkyl ist oder R und R₁ zusammen eine -CH=N-CH₂-Gruppe bilden.

5. Verbindung nach Anspruch 3 oder 4, worin R₂ Pyridyl ist, R = Wasserstoff oder Halogen ist, R₃ und R₄ unabhängig voneinander Wasserstoff oder Methyl bedeuten, R₁ Wasserstoff ist oder R und R₁ zusammen eine -CH=N-CH₂-Gruppe bilden.

6. Verbindung nach einem der Ansprüche 1 bis 5, d. h.
2-(4-Pyridyl)thiazolo[5,4-g]-4-chlor-7,8-dihydro-6(5H)chinolon,
2-(4-Pyridyl)thiazolo[5,4-g]-4-brom-7,8-dihydro-6-(5H)chinolon,
2-(4-Pyridyl)thiazolo[4,5-f]-6(7H)-chinolon,
2-(1-Oxido-4-pyridyl)thiazolo[5,4-g]-4,8,8-trimethyl-7,8-dihydro-6(5H)chinolon,
2-(3-Pyridyl)thiazolo[5,4-g]-7,8-dihydro-6(5H)chinolon,
2-(4-Pyridyl)thiazolo[5,4-g]-5-methyl-7,8-dihydro-6(5H)chinolon,
2-(4-Pyridyl)thiazolo[5,4-g]-8-methyl-6(5H)-chinolon-trifluoracetat,
2-(4-Pyridyl)thiazolo[5,4-g]-7-methyl-7,8-dihydro-6(5H)chinolon-hydrobromid-methanolat,
2-(4-Pyridyl)-5,5-dimethyl-5H-thiazolo[4,5-g]-7[8H)chinolon-hydrobromid,
2-(4-Pyridyl)-9,9-dimethyl-6H-thiazolo[5,4-f]-7[8H)chinolon-hydrobromid,
2-(4-Pyridyl)-8-methyl-7,8-dihydrothiazolo[5,4-g]-6(5H)chinolon-trifluoracetat,
2-(4-Pyridyl)-4,8,8-trimethyl-7,8-dihydrothiazolo[5,4-g]-6(5H)-chinolon,
2-(2-Pyridyl)thiazolo[5,4-g]-7,8-dihydro-6(5H)-chinolon,
2-(2-Amino-4-pyrimidinyl)thiazolo[5,4-g]-7,8-dihydro-6(5H)chinolon-trifluoracetat,
2-(4-Morpholinyl)thiazolo[5,4-g]-7,8-dihydro-6(5H)chinolon,
2-(3,4,5-Trimethoxyphenyl)thiazolo[5,4-g]-7,8-dihydro-6(5H)chinolon,
Triazolo[5,4-g]-7,8-dihydro-6(5H)chinolon oder
2-(4-Pyridyl)thiazolo[5,4-g]-7,8-dihydro-6(5H)-chinolon.

7. Zusammensetzung zur Behandlung von dekompensierter Herzinsuffizienz, wobei die genannte Zusammensetzung einen pharmazeutisch verwendbaren inerten Träger und - als aktive Komponente derselben - eine wirksame Menge einer Verbindung nach einem der Anspüche 1 bis 6 umfaßt.

## Revendications

1. Composés de formule : 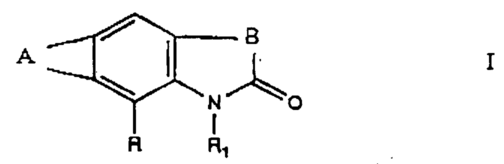 et sels pharmaceutiquement acceptables de ceux-ci, dans laquelle A représente le groupe -N=N-NH-, -S-C(R₂)=N-, ou -N=C(R₂)-S- dans lequel R₂ est un groupe amino, pyridyle, pyrimidinyle, aminopyrimidinyle, oxydopyridyle, morpholinyle, ou triméthoxyphényle, B est un groupe -(CH₂)ₘ-C(R₃R4)-(CH₂)ₙ- dans lequel R₃ et R₄ sont indépendamment, un atome d'hydrogène ou un groupe alkyle C₁₋₆, et m et n sont différents l'un de l'autre, et égaux à 0 ou à 1, R est un atome d'hydrogène, un groupe alkyle C₁₋₆, un groupe amino, ou un atome d'halogène, R₁ est un atome d'hydrogène, ou un groupe alkyle C₁₋₆, ou R et R₁ ensemble, forment un groupe -CH=N-CH₂-.

2. Composés de formule : 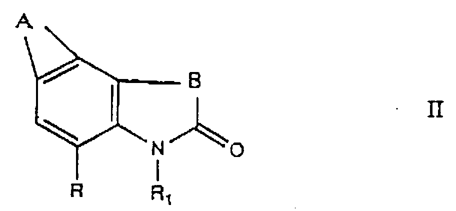 et sels pharmaceutiquement acceptables de ceux-ci, dans laquelle A représente le groupe -N=N-NH-, -S-C(R₂)=N-, ou -N=C(R₂)-S- dans lequel R₂ est un groupe amino, pyridyle, pyrimidinyle, aminopyrimidinyle, oxydopyridyle, morpholinyle, ou triméthoxyphényle, B est un groupe -(CH₂)ₘ-C(R₃R₄)-(CH₂)ₙ- dans lequel R₃ et R₄ sont indépendamment, un atome d'hydrogène ou un groupe alkyle C₁₋₆, et m et n sont différents l'un de l'autre, et égaux à 0 ou à 1, R est un atome d'hydrogène, un groupe alkyle C₁₋₆, un groupe amino, ou un atome d'halogène, R₁ est un atome d'hydrogène, ou un groupe alkyle C₁₋₆, ou R et R₁ ensemble, forment un groupe -CH=N-CH₂-.

3. Composé selon la revendication 1, ayant la formule : 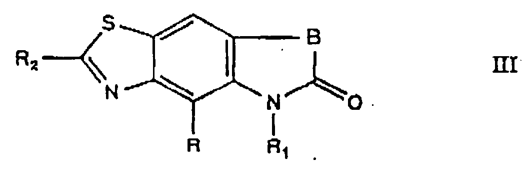 dans laquelle R₂ est un groupe amino, pyridyle, pyrimidinyle, aminopyrimidinyle, oxydopyridyle, morpholinyle, ou triméthoxyphényle, B est un groupe -(CH₂)ₘ-C(R₃R₄)-(CH₂)ₙ- dans lequel R₃ et R₄ sont indépendamment, un atome d'hydrogène ou un groupe alkyle C₁₋₆, et m et n sont différents l'un de l'autre, et égaux à 0 ou à 1, R est un atome d'hydrogène, un groupe alkyle C₁₋₆, un groupe amino, ou un atome d'halogène, R₁ est un atome d'hydrogène, ou un groupe alkyle C₁₋₆, ou R et R₁ ensemble, forment un groupe -CH=N-CH₂-.

4. Composé selon la revendication 3, ayant la formule : 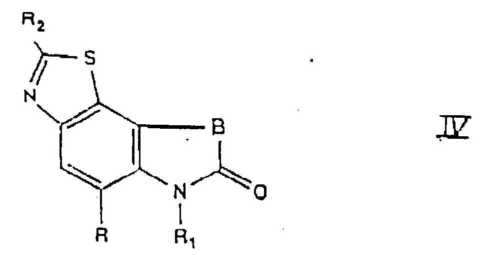 dans laquelle R₂ est un groupe amino, pyridyle, pyrimidinyle, aminopyrimidinyle, oxydopyridyle, morpholinyle, ou triméthoxyphényle, B est un groupe -(CH₂),ₙ-C(R₃R4)-(CH₂)ₙ- dans lequel R₃ et R₄ sont indépendamment, un atome d'hydrogène ou un groupe alkyle C₁₋₆, et m et n sont différents l'un de l'autre, et égaux à 0 ou à 1, R est un atome d'hydrogène, un groupe alkyle C₁₋₆, un groupe amino, ou un atome d'halogène, R₁ est un atome d'hydrogène, ou un groupe alkyle C₁₋₆, ou R et R₁ ensemble, forment un groupe -CH=N-CH₂-.

5. Composé selon la revendication 3, ou la revendication 4, dans lequel R₂ est un groupe pyridyle, R est un atome d'hydrogène, ou un atome d'halogène, R₃ et R₄ sont indépendamment un atome d'hydrogène ou un groupe méthyle, R₁ est un atome d'hydrogène, ou R et R₁ ensemble, forment un groupe -CH=N-CH₂-.

6. Composé selon l'une quelconque des revendications 1 à 5, qui est :
- la 2-(4-pyridyl)thiazolo[5,4-g]4-chloro-7,8-dihydro-6(5H)quinolone,
- la 2-(4-pyridyl)thiazolo[5,4-g]-4-bromo-7,8-dihydro-6(5H)quinolone,
- la 2-(4-pyridyl)thiazolo[5,4-f]-6(7H)-quinolone,
- la 2-(1-oxydo-4-pyridyl)thiazolo[5,4-g]-4,8,8-triméthyl-7,8-dihydro-6(5H)quinolone,
- la 2-(3-pyridyl)thiazolo[5,4-g]-7,8-dihydro-6(5H)-quinolone,
- la 2-(4-pyridyl)thiazolo[5,4-g]-5-méthyl-7,8-dihydro-6(5H)-quinolone,
- le 2-(4-pyridyl)thiazolo[5,4-g]-8-méthyl-6(5H)-quinolone trifluoroacétate,
- le -(4-pyridyl)thiazolo[5,4-g]-7-méthyl-7,8-dihydro-6(5H)-quinolone bromhydrate méthanolate
- le 2-(4-pyridyl)-5,5-diméthyl-5H-thiazolo[4,5-g]-7(8H)-quinolone bromhydrate,
- le 2-(4-pyridyl)-9,9-diméthyl-6H-thiazolo[5,4-f]-7(8H)quinolone bromhydrate,
- le 2-(4-pyridyl)-8-méthyl-7,8-dihydrothiazolo[5,4-g]-6(5H)quinolone trifluoroacétate,
- la 2-(4-pyridyl)-4,8,8-triméthyl-7,8-dihydrothiazolo-[5,4-g]-6(5H)quinolone,
- la 2-(2-pyridyl)thiazolo[5,4-g]-7,8-dihydro-6(5H)-quinolone,
- le 2-(2-amino-4-pyrimidinyl)thiazolo[5,4-g]-7,8-dihydro-6(5H)quinolone trifluoroacétate,
- la 2-(4-morpholinyl)thiazolo[5,4-g]-7,8-dihydro-6(5H)-quinolone,
- la 2-(3 ,4,5-triméthoxyphényl)thiazolo[5,4-g]-7,8-dihydro-6(5H)quinolone,
- la triazolo[5,4-g]-7,8-dihydro-6(5H)-quinolone, ou
- la 2-(4-pyridyl)thiazolo[5,4-g]-7,8-dihydro-6(5H)quinolone,

7. Composition pour le traitement de la défaillance cardiaque congestive, ladite composition comprenant un véhicule inerte pharmaceutiquement acceptable, et en tant que composant actif, une quantité efficace d'un composé selon l'une quelconque des revendications 1 à 6.
